(19) Europäisches Patentamt
European Patent Office
Office européen des brevets

(11) Publication number: 0 431 884 A2

(12) EUROPEAN PATENT APPLICATION

(21) Application number: 90313134.0

(51) Int. Cl.$^5$: A61K 31/77

(22) Date of filing: 04.12.90

(30) Priority: 05.12.89 US 446276

(43) Date of publication of application:
12.06.91 Bulletin 91/24

(84) Designated Contracting States:
AT BE CH DE DK ES FR GB GR IT LI LU NL SE

(71) Applicant: CALGON CORPORATION
Route 60-Campbell's Run Road
Robinson Township Pennsylvania 15205 (US)

(72) Inventor: Brenden, Rita A.
808 S. Brentwood Blvd., Apt. 3B
Clayton, Missouri 63105 (US)
Inventor: Burkey, Jennifer L.
2224 Ben Clare Drive
Fenton, Missouri 63026 (US)
Inventor: Kirchner, Fred T.
No. 6 Oakwood Drive
St. Charles, Missouri 63301 (US)

(74) Representative: Hesketh, Alan, Dr. et al
European Patent Department Merck & Co., Inc. Terlings Park Eastwick Road
Harlow Essex, CM20 2QR (GB)

(54) Non-toxic wound cleansing compositions.

(57) Aqueous wound cleansing compositions containing at least one non ionic ethylene oxide/propylene oxide block copolymer and at least one physiological salt and the use thereof to cleanse and/or treat wounds in a non-toxic manner.

EP 0 431 884 A2

# NON-TOXIC WOUND CLEANSING COMPOSITIONS

## BACKGROUND OF THE INVENTION

U.S. Patent Re. 29,909 to Kurtz discloses a method of cleansing wounds which utilizes aqueous detergent solutions of ethylene oxide (EO)/propylene oxide (PO) block copolymers. The block copolymers of Kurtz are commercially available as Pluronic$^R$ Polyols from BASF. Kurtz discloses that his designated EO/PO polymers do not impair the ability of a wound being treated to resist infection. The claimed Kurtz wound cleansing compositions consist of an aqueous detergent solution of at least about 10% of an EO/PO block copolymer having an EO/PO ratio of at least 3 : 1 and a molecular weight of from about 5,000 to about 15,000.

A wound cleanser known as Shur Clens$^R$, which is commercially available from Calgon-Vestal Laboratories, represents a commercial embodiment of a wound cleansing composition disclosed by Kurtz.

In general, the use of detergent surfactants for wound cleansing is avoided because such detergents delay the healing of wounds. This delay is thought to be due to the effects of detergent surfactants on cellular components in wounds. These components include, but are not limited to, white blood cells, red blood cells, and fibroblasts. The detergent surfactants would otherwise be preferred because of their ability to remove exudate, dried blood and slough from the wound. It is noteworthy that detergents may be toxic in a wound cleansing sense (that is, cytotoxic) even though they would be considered non-toxic by other standards of measurement, for instance, oral toxicity, eye irritation, and skin sensitivity. Thus, a number of detergents classified as non-toxic on the basis of other studies unfortunately prove to be toxic in wound healing in that they are cytotoxic.

Among the commercially available products that are used to cleanse and/or treat wounds are Hibiclens$^R$, Betadine$^R$, and pHisohex$^R$. The harmful effects of such products have been reported and confirmed in studies by Lineaweaver, Howard, Soucy, McMorris, Freeman, Crain, Robertson, and Rumley ("Topical Antimicrobial Toxicity", Archives of Surgery, 120 : 267-270, 1985) and by Bryant, Rodeheaver, Reem, Nichter, Keeney, and Edlich ("Search for a Nontoxic Surgical Scrub Solution for Periorbital Lacerations", Annals of Emergency Medicine, 13 : 317-321, May, 1984).

As a consequence of the toxic effects of detergents on wounds, physicians today oftentimes simply irrigate wounds with large amounts of 0.9% sodium chloride solution.

## SUMMARY OF THE INVENTION

The instant inventors have surprisingly discovered that the toxic effects of selected detergent surfactants are reduced and possibly eliminated by the addition of an effective amount of at least one physiological salt to such toxic detergent surfactants.

Thus, the exceptional detergency qualities of EO/PO surfactant polymers previously thought to be toxic as wound cleansers at useful concentrations can now be used to full advantage in wound cleansing compositions because their wound cleansing toxicity is reduced or possibly eliminated by the presence of a physiological salt in wound cleansing compositions containing such detergent surfactants.

For example, while 2% (by weight) aqueous solutions of various nonionic EO/PO block copolymer surfactants produced good cleaning of dried blood, these formulations had an adverse effect (cytotoxicity) when placed in contact with mouse fibroblast cells in-vitro for prolonged exposure times. By contrast, solutions containing 0.85% (by weight) NaCl and 2% (by weight) of the same nonionic EO/PO polymer surfactants surprisingly produced good cleaning of dried blood and eliminated or markedly reduced the toxicity response in-vitro. This discovery enables wound cleansing compositions to be prepared which can be used in the healthcare environment without adversely affecting the patient's wound. Such compositions provide excellent cleansing action without manifesting the toxic wound cleansing side effects common to detergent surfactants.

One object of the instant invention is therefore to provide a non-toxic detergent surfactant composition which can be used for various wound management purposes, including but not limited to wound cleansing, irrigation and soaking purposes, without substantial toxic side effects.

Most commercial wound cleansers are intended for use as cleansing or irrigating solutions with minimal or short term exposure to tissues. Wound cleansers usually contain strong anionic or amphoteric surfactants in low concentrations to give good cleaning. However, these cleansers lyse or kill the tissue cells in in-vitro cell cultures usually within fifteen minutes. Normal saline has no cleansing action but is a commonly used irrigation fluid in the field of wound management. The instant compositions differ in that they cleanse and can remain in contact with wounds for extended periods of time without causing substantial cytotoxicity.

Healthcare providers sometimes use saline to soak wounds, i.e., they spray gauze pads with saline and then place these wet pads on the wound for up to 8 hours. Traditional wound cleansers would not be satisfactory

for this application because of their toxicity to wound tissue. For this reason, the wound management community has long sought a wound cleansing product suitable for short or long term exposure to wound tissues. The instant invention relates to such compositions, which combine cleaning action with nontoxicity for short or long term applications in treating wounds.

## DETAILED DESCRIPTION OF THE INVENTION

The instant invention is directed to a non-toxic composition for treatment of wounds and/or cleansing comprising :

(a) an effective amount, preferably about 0.001% to about 40%, by weight of the composition, of at least one nonionic, water soluble surfactant polymer selected from the group of ethylene oxide/propylene oxide block copolymers defined as :

(I)

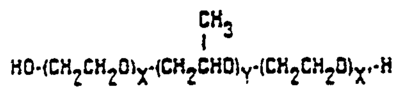

X, X' = 2-122 ; Y = 16-54

(II)

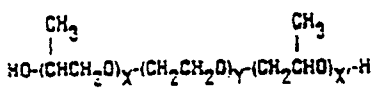

X, X' = 7-21 ; Y = 4-163

(III)

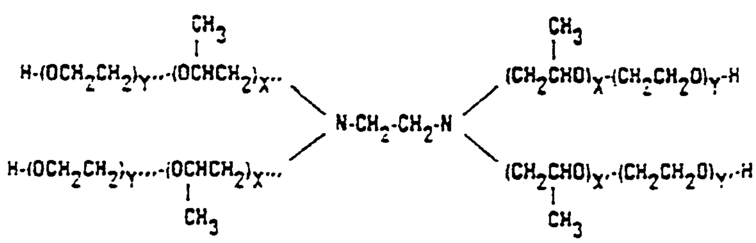

X, X', X" X''' = 2-122 ; Y, Y', Y", Y''' = 2-32

or (IV)

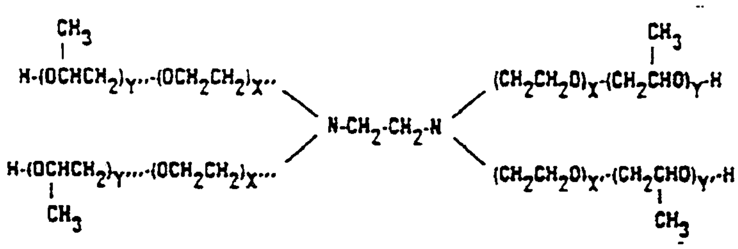

X, X', X", X''' = 2-122 ; Y, Y', Y", Y''' = 2-32
or mixtures thereof ;

(b) an effective amount of at least one physiological salt, or mixtures of physiological salts ; and

(c) the balance water.

The instant invention is also directed to the use of the above compositions in wound management for the treatment of wounds, wherein they contact wounds for purposes which include but are not limited to wound cleansing, wound irrigation and wound soaking.

The instant invention is further directed to a method for lowering the cytotoxicity of the above listed nonionic water-soluble surfactant polymers by adding an effective amount for the purpose of at least one physiological salt to these polymers in aqueous solution.

The component (a) surfactant polymers are preferably block copolymers of ethylene oxide (EO) and proylene oxide (PO). More particularly, the polymers of structure (I) comprise poly(oxyethylene) groups at both ends of a poly(oxypropylene) chain, the polymers of structure (II) comprise poly(oxypropylene) groups at both ends of a poly(oxyethylene) chain, the polymers of structure (III) comprise PO-EO capped ethylene diamines and the polymers of structure IV comprise EO-PO capped ethylenediamines. Examples of such copolymer surfactants include products sold under the tradenames Pluronic (class I), Pluronic R (class II), Tetronic (class III), and Tetronic R (class IV), which are commercially available from BASF Corporation. Methods for preparing such polymers are well known in the art.

The more preferred surfactant polymers are the polymers of type II, and the most preferred surfactant polymers are those of type II where X and X' equal about 7 and Y equals about 22.

Preferably, component (a) comprises about 0.001% to about 40% by weight of the instant compositions, more preferably about 0.1% to about 20% and most preferably about 0.5 to about 5%.

The component (a) surfactants of the instant invention must be water-soluble at use concentrations, and preferably exhibit a solubility in water of greater than about 10 grams per 100 ml. The cleansing compositions described herein may be prepared by simply dissolving at least one component (a) polymer in water, followed by addition of an effective amount of at least one physiological salt. The concentration of polymer in the water may vary according to the strength of the detergency sought and the desired viscosity. Minimally, the EO/PO surfactant polymer must be present in an effective amount, that is, at a sufficient concentration to effect treatment and/or cleansing of the wound being treated. In general, aqueous solutions containing in the range of 0.5 to 5% active polymer are most preferred.

Any physiological salt can be used as component (b). As used herein, a physiological salt is a salt which, when in solution at a concentration, by weight, of less than or equal to about 2.0%, does not substantially alter the function or integrity of the cells of a wound in contact with the salt solution. Examples of physiological salts include alkaline and alkaline earth metal chlorides, phosphates and sulfates. Preferred physiological salts include KCl, NaCl, $MgCl_2$, $CaCl_2$, and mixtures thereof. The most preferred physiological salt is NaCl.

An effective amount of the salt should be used. As used herein, the term effective amount refers to that amount of salt necessary to lower the toxicity of the surfactant(s) or surfactant solution to which it is added. Generally, at least about 0.01% by weight, based on total composition weight, is required. Preferably, the salt concentration in the instant compositions will range from about 0.1 to 1.75 weight %, most preferably from about 0.2 to 1.3 weight %, based on total composition weight. In no instance, however, should the salt concentration exceed about 2.0%, by weight, of the total composition. At higher concentrations, the salt itself is generally toxic to wound tissues.

If desired, the instant compositions may also include other materials commonly employed in wound cleansing solutions. For example, effective amounts for the purpose of preservatives, dyes, emollients, perfumes, sugars, proteins, vitamins, minerals, glycols and/or alcohols can be added. Additionally, any known antiseptic or antimicrobial can optionally be added. Such antiseptic or antimicrobial agents include, but are not limited to, ethyl alcohol, benzalkonium chloride, chlorhexidine gluconate, iodine, iodophors such as polyvinylpyrrolidone/iodine, and the like.

The balance of the instant compositions is water. Of course, relatively pure water, for example, USP purified water, is preferred.

The instant invention also relates to methods of treating wounds comprising contacting a wound with the instant compositions for the purpose of cleansing, irrigating and/or soaking the wound. Any known technique for contacting a wound with the instant compositions may be employed, including but not limited to swabbing or scrubbing with gauze, sponges, surgical cotton and the like moistened with the instant cleansing solution, soaking of the wound in the instant composition, hypodermic flushing of the wound using the instant composition and pouring or spraying the instant composition onto a wound.

The key to the above described inventions is the surprising discovery by the inventors that effective amounts of one or more physiological salts reduce and possibly eliminate the toxic effects of various detergent surfactants on wound cells. Thus, while uses of the surfactant components (a) and the physiological salt components (b) are individually known in the art, the synergisitic interaction between these components relative to cell toxicity is not known or suggested in the art.

## EXAMPLES

## EXAMPLES 1-48 - CYTOTOXICITY SCREENING

The following examples further demonstrate the instant invention. These examples are not intended to limit the invention in any way.

Methodology

L929 mouse fibroblasts, an established cell line available from American Type Culture Collection (ATCC), were grown to confluence in tissue culture dishes. A test material (the wound cleansing composition) was placed in direct contact with the cell monolayer for a designated amount of time (15 minutes, 1 hour, or 4 hours) by pipetting the test material on to the fibroblasts. After the contact period, the composition was removed by pouring it from the dishes containing the fibroblasts, and a 0.002% fluorescein diacetate dye solution was added to the dish to cover the cell monolayer. Only those cells which were still viable absorbed the dye. The percentage of undamaged cells was determined visually by observation with a fluorescent microscope. Each observation is a summary of two (2) replicates.

Materials

The following surfactants and physiological salts were tested :
Pluracare 10R5 is a block copolymer of type II, with an approximate composition of X, X′ = 7 ; y = 22 manufactured by BASF Corporation.
Pluronic F68 is a block copolymer of type I, with an approximate composition of X, X′ = 85 ; y = 30 manufactured by BASF Corporation.
Tetronic 304 is a block copolyner of type III, with an approximate composition of X, X′, X″, X‴ = 4 ; y, y′, y″, y‴ = 3 manufactured by BASF Corporation.
Pluronic P-104 is a block copolymer of type I, with an approximate composition of X, X′ = 31 ; y = 54 manufactured by BASF Corporation.
Pluronic L64 is a block copolymer of type I, with X, X′ = 13 ; y = 30 manufactured by BASF Corporation.
Tetronic 904 is a block copolymer of type III, with X, X′, X″, X‴ = 19 ; y, y′, y″, y‴ = 16 manufactured by BASF Corporation.
Tetronic 50R4 is a block copolymer of type IV, with X, X′, X″ and X‴ = 9 ; Y, Y′, Y″ and Y‴ = 10 manufactured by BASF Corporation.
Tergitol 15S9 is a Pareth 9* manufactured by Union Carbide Corporation.
Surfonic N95 is a nonoxynol-9* manufactured by Jefferson Chemical.
Miranol C2M is cocoamphocarboxyglycinate* manufactured by Miranol Corporation.
Glydant is a solution of dimethylol-dimethyl-hydantoin manufactured by Lonza Corporation.
Mackanate ELB is disodium laureth sulfosuccinate* manufactured by MacIntyre Chemical Corporation.
Cyclomox L is lauramine oxide * manufactured by Cyclo Chemical Corporation.
Sodium Alpha Olefin Sulfonate, available from Witco Chemical Company.
Ringer's solution is a mixture of 0.86% NaCl, 0.03% KCl and 0.036% $CaCl_2$ in water.
Tyrode's solution is a mixture of 0.8% NaCl, 0.02% KCl, 0.02% $CaCl_2$, 0.005% $MgCl_2$, 0.003% $NaH_2PO_4$, 0.1% dextrose and 0.1% $NaHCO_3$ in water.
Hepes is a 1.0 M solution of (4-(2-hydroxyethyl)1-piperazinethanesulfonic acid).

*CTFA designated name.

Results

Examples 1-14 in Table I show that the toxicity of various block copolymers is lowered in the presence of saline (0.85% sodium chloride by weight). All surfactants were present at a 2% by weightconcentration. The results in the table show each surfactant in water only and with the added salt. The results show the percent viability of the fibroblast cells after exposure times of 15 minutes, 1 hour, and 4 hours.

Examples 15-21 in Table II show other classes of surfactants. All were tested at a 2% weight concentration in the presence of saline (0.85% sodium chloride). All were cytotoxic under these conditions.

Examples 22-31 in Table III show tests with levels of a selected surfactant from 0.175% to 3.5%. The selected surfactant was Pluracare 10R5. These levels were run both with and without saline (0.85% sodium chloride).

Examples 32-40 in Table IV illustrate the use of different physiological salts, all used with Pluracare 10R5 at a weight concentration of 2%. The results show that the salts in examples 32-37 had a beneficial effect, while those in examples 38-40 did not.

Examples 41-45 in Table V illustrate the use of one salt (sodium chloride) at differing concentrations when

used with 2% of Pluracare 10R5.

Examples 46-48 in Table VI show that other additives, such as preservatives, can be used. These examples demonstrate the use of 2% Pluracare 10R5, with 0.85% sodium chloride, and optionally with two (2) preservatives.

An example of a most preferred compositions comprises :
Pluracare 10R5 - 2.0% by weight ;
NaCl - 0.85% by weight ; and
USP water - balance.

Table I

| Example | Surfactant class | Surfactant | Saline | % Viability 15 minutes | % Viability 1 hour | % Viability 4 hours |
|---|---|---|---|---|---|---|
| 1 | I | Pluronic F-68 | No | 90 | 40 | 10 |
| 2 | I | Pluronic F-68 | Yes | 100 | 100 | 100 |
| 3 | I | Pluronic 104 | No | 20 | 30 | 5 |
| 4 | I | Pluronic 104 | Yes | 100 | 100 | 95 |
| 5 | I | Pluronic L-64 | No | 80 | 3 | 0 |
| 6 | I | Pluronic L-64 | Yes | 100 | 25 | 0 |
| 7 | II | Pluracare 10R-5 | No | 90 | 85 | 30 |
| 8 | II | Pluracare 10R-5 | Yes | 100 | 100 | 100 |
| 9 | III | Tetronic 304 | No | 95 | 95 | 50 |
| 10 | III | Tetronic 304 | Yes | 100 | 100 | 100 |
| 11 | III | Tetronic 904 | No | 85 | 70 | 10 |
| 12 | III | Tetronic 904 | Yes | 100 | 95 | 20 |
| 13 | IV | Tetronic 50R-4 | No | 90 | 90 | 70 |
| 14 | IV | Tetronic 50R-4 | Yes | 100 | 100 | 100 |

Table II

| Example | Surfactant | Saline | % Viability 15 minutes | % Viability 1 hour | % Viability 4 hours* |
|---|---|---|---|---|---|
| 15 | Tergitol 15S9 | Yes | 0 | 0 | 0 |
| 16 | Surfonic N95 | Yes | 0 | 0 | - |
| 17 | Sodium Lauryl Sulfate | Yes | 0 | 0 | - |
| 18 | Miranol C2M | Yes | 0 | 0 | - |
| 19 | Cyclomox L | Yes | 0 | 0 | - |
| 20 | Mackanate ELB | Yes | 0 | 0 | - |
| 21 | Sodium Alpha Olefin Sulfonate | Yes | 0 | 0 | - |

*Due to lack of viability at shorter time intervals, the four hour reading was not done in most cases.

Table III

| Example | Percent Surfactant | Saline | % Viability 15 minutes | 1 hour | 4 hours |
|---|---|---|---|---|---|
| 22 | 0.175% | No | 90 | 30 | 15 |
| 23 | 0.175% | Yes | 95 | 95 | 95 |
| 24 | 0.5% | No | 75 | 60 | 10 |
| 25 | 0.5% | Yes | 100 | 100 | 100 |
| 26 | 1% | No | 75 | 75 | 10 |
| 27 | 1% | Yes | 100 | 100 | 100 |
| 28 | 2% | No | 75 | 50 | 90 |
| 29 | 2% | Yes | 100 | 100 | 95 |
| 30 | 3.5% | No | 95 | 90 | 95 |
| 31 | 3.5% | Yes | 100 | 100 | 95 |

Table IV

| Example | Salt | Percent Solids | % Viability 15 minutes | 1 hour | 4 hours |
|---|---|---|---|---|---|
| 32 | NaCl | 0.85% | 100 | 95 | 95 |
| 33 | $MgCl_2$ | 0.85% | 100 | 95 | 95 |
| 34 | $CaCl_2$ | 0.85% | 100 | 95 | 80 |
| 35 | KCl | 0.85% | 100 | 100 | 70 |
| 36 | Ringer's solution | 0.926%* | 100 | 100 | 95 |
| 37 | Tyrode's solution | 1.05%** | 100 | 100 | 95 |
| 38 | Hepes | 23.83%*** | 100 | 70 | 0 |
| 39 | Sodium Bicarbonate | 7.5% | 90 | 0 | 0 |
| 40 | Glycine | 7.5% | 100 | 10 | 0 |

*Consists of:

| | |
|---|---|
| $CaCl_2$ | 0.036% |
| KCl | 0.030% |
| NaCl | 0.860% |
| Total solids: | 0.926% |

**Consists of:

| | |
|---|---|
| NaCl | 0.800% |
| KCl | 0.020% |
| $CaCl_2$ | 0.020% |
| $MgCl_2$ | 0.005% |
| $NaH_2PO_4$ | 0.003% |
| $NaHCO_3$ | 0.100% |
| Dextrose | 0.100% |
| Total solids: | 1.05% |

***Consists of:

N-2-Hydroxyethylpiperazine-N'-2-ethane sulfonic acid 1 M

Total solids: 23.83%

Table V

| Example | Percent NaCl | % Viability | | |
|---|---|---|---|---|
| | | 15 minutes | 1 hour | 4 hours |
| 41 | 0.1% | 100 | 100 | 95 |
| 42 | 0.85% | 100 | 100 | 100 |
| 43 | 1% | 100 | 100 | 70 |
| 44 | 1.65% | 100 | 100 | 80 |
| 45 | 2% | 100 | 95 | 45 |

Table VI

| Example | Percent NaCl | Percent Glydant | Percent Potassium Sorbate | % Viability | | |
|---|---|---|---|---|---|---|
| | | | | 15 minutes | 1 hour | 4 hours |
| 46 | 0.85% | 0% | 0% | 100 | 100 | 100 |
| 47 | 0.85% | 0.1% | 0.1% | 100 | 100 | 90 |
| 48 | 0% | 0.1% | 0.1% | 100 | 80 | 5 |

## Claims

1. A composition comprising :

(a) an effective amount of a nonionic, water soluble surfactant polymer selected from the group consisting of ethylene oxide/propylene oxide block copolymers defined as :

(I)

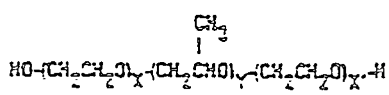

X, X′ = 2-122 ; Y = 16-54

(II)

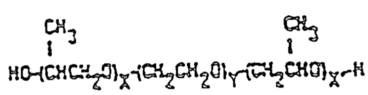

X, X′ = 7-21 ; Y = 4-163

(III)

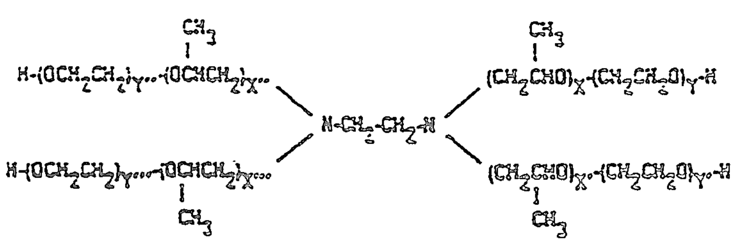

X, X′, X″, X‴ = 2-122 ; Y, Y′, Y″, Y‴ = 2-32

or (IV)

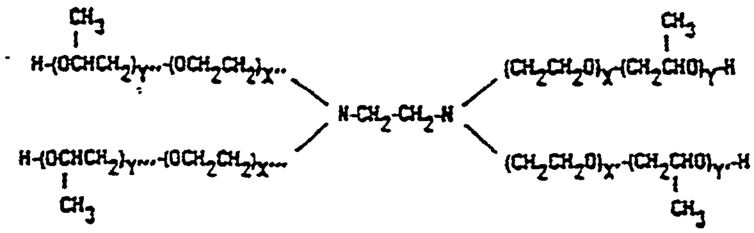

X, X', X'', X''' = 2-122 ; Y, Y', Y'', Y''' = 2-32
(b) an effective amount of a physiological salt ; and
(c) the balance water.

2. The composition of Claim 1 which comprises about 0.001 to about 40 percent, by weight, of said surfactant polymer and about 0.01 to about 2.0 percent, by weight, of said physiological salt.

3. The composition of Claim 2, wherein said physiological salt is selected from the group consisting of alkaline and alkaline earth metal chlorides, phosphates and sulfates.

4. The composition of Claim 3, wherein said surfactant polymer is of type II and wherein said physiological salt is selected from the group consisting of KCl, NaCl, $MgCl_2$, $CaCl_2$ and mixtures thereof.

5. The composition of Claim 4 which comprises about 0.1 to about 20.0 percent of said surfactant polymer and about 0.1 to about 1.75 percent of said, physiological salt.

6. The composition of Claim 5 wherein X and X' of said surfactant polymer are about 7, wherein Y of said surfactant polymer is about 22 and wherein said physiological salt is sodium chloride.

7. The use, for the manufacture of a medicament for treating a wound, of a composition comprising :
(a) an effective amount of a nonionic, water soluble surfactant selected from the group consisting of ethylene oxide/propylene oxide block copolymers defined as :

(I)

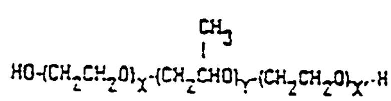

X, X' = 2-122 ; Y = 16-54

(II)

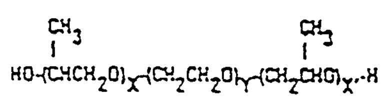

X, X' = 7-21 ; Y = 4-163

(III)

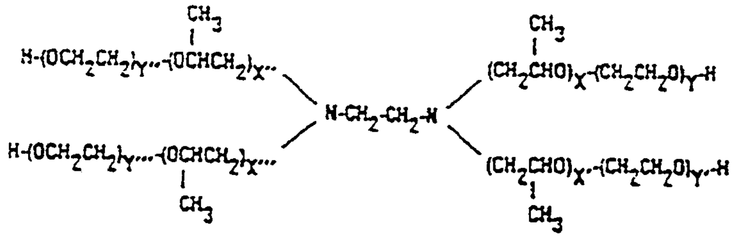

X, X′, X″, X‴ = 2-122 ; Y, Y′, Y″, Y‴ = 2-32

or. (IV) 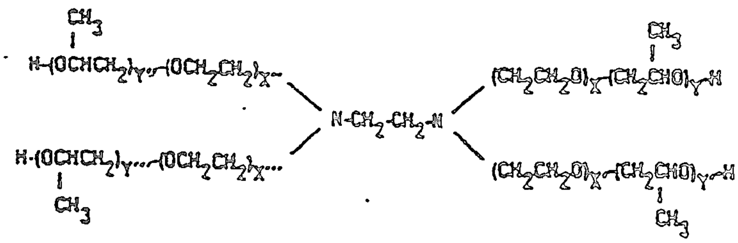

X, X′, X″, X‴ = 2-122 ; Y, Y′, Y″, Y‴ = 2-32
(b) an effective amount of a physiological salt ; and
(c) the balance water.

8. The use as claimed in Claim 7 wherein said composition comprises about 0.001 to about 40%, by weight, of said surfactant polymer and about 0.01 to about 2.0 percent, by weight, of said physiological salt.

9. A method for lowering the cell toxicity of an aqueous solution containing about 0.001 to about 40 percent, by weight, of a water soluble surfactant polymer selected from the group consisting of ethylene oxide/propylene oxide block copolymers defined as :

(I)

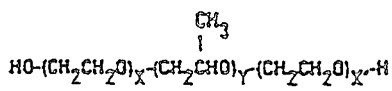

X, X′ = 2-122 ; Y = 16-54

(II)

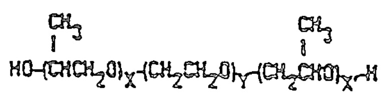

X,X′ = 7 - 21; y = 4 - 163

X, X′ = 7-21 ; Y = 4-163

(III)

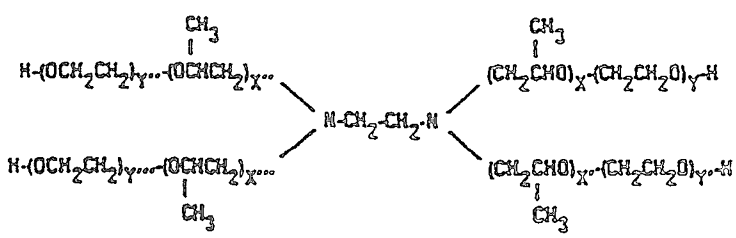

X, X′, X″, X‴ = 2-122 ; Y, Y′, Y″, Y‴ = 2-32

or (IV)

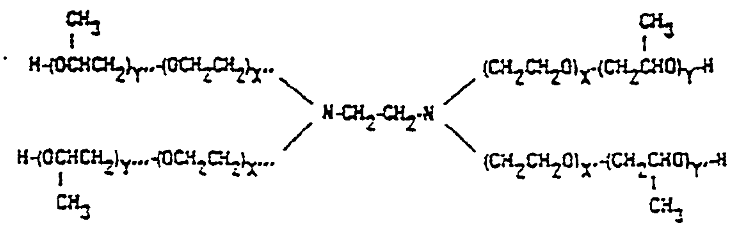

X, X′, X″, X‴ =2-122 ; Y, Y′, Y″, Y‴ = 2-32
comprising adding to said aqueous solution an effective amount of a physiological salt.

**10.** The method of Claim 9 wherein said effective amount of said physiological salt is about 0.01 to about 2.0%, by weight, of said aqueous solution.